# FASCICULE DE BREVET EUROPEEN

(11) **EP 1 133 979 B1**
(45) Date de publication et mention de la délivrance du brevet: **26.10.2005**
(21) Numéro de dépôt: 01400307.3
(22) Date de dépôt: 08.02.2001
(51) Int. Cl.: A61K 7/32, A61K 7/48

(54) **Utilisation de corps gras particuliers permettant de modifier les propriétés physico-chimiques de la peau et/ou des muqueuses en tant qu'agents empêchant ou diminuant l'adhésion des micro-organismes sur ces dernières**
Verwendung von spezifischen Fettstoffen zur Modifizierung der physico-chemischen Eigenschaften der Haut und/oder der Schleimhaut als Mittel zur Verhinderung oder Reduzierung der Adhäsion von Mikro-organismen darauf
Use of specific fatty compounds for modifying the physico-chemical properties of the skin and/or the mucous membrane as agents for preventing or reducing the adhesion of micro-organisms thereon

(30) Priorité: 15.02.2000 FR 0001842
(43) Date de publication de la demande: 19.09.2001
(73) Titulaire: L'OREAL, 75008 Paris (FR)
(72) Inventeur: Lerebour, Géraldine, 91200 Athis-Mons (FR); Arnaud-Sebillotte, Laurence, 94240 L'Hay les Roses (FR)
(74) Mandataire: Casalonga, Axel

(56) Documents cités:
- EP-A- 0 315 912
- EP-A- 0 613 694
- EP-A- 0 697 213
- EP-A- 0 875 239
- WO-A-95/31956
- CH-A- 688 787
- DE-A- 4 134 137
- DE-A- 4 330 664
- DE-A- 19 503 423
- DE-A- 19 634 021
- DE-A- 19 634 959
- DE-A- 19 643 585
- FR-A- 2 689 011
- FR-A- 2 768 925
- US-A- 4 172 149
- US-A- 4 451 480
- US-A- 5 359 774
- YOUSEF, N. E. ET AL: "Inhibition of bacterial adherence and biofilm on contact lenses" EGYPT. J. BIOMED. SCI. (1998), 1, 79-94, 1998, XP002158695
- PATENT ABSTRACTS OF JAPAN vol. 017, no. 617 (C-1129), 15 novembre 1993 (1993-11-15) & JP 05 186328 A (KAO CORP), 27 juillet 1993 (1993-07-27)
- PATENT ABSTRACTS OF JAPAN vol. 016, no. 207 (C-0941), 18 mai 1992 (1992-05-18) & JP 04 036221 A (SUNSTAR INC), 6 février 1992 (1992-02-06)

## Description

L'invention concerne l'utilisation de corps gras particuliers permettant de modifier les propriétés physico-chimiques de la surface de la peau et/ou des muqueuses dans une composition cosmétique ou pour la préparation d'une composition pharmaceutique en tant qu'agents empêchant ou diminuant l'adhésion des micro-organismes, particulièrement des bactéries, sur la peau et/ou les muqueuses.

La peau humaine est peuplée en permanence d'une multitude de micro-organismes différents (bactéries, levures et champignons). La flore microbienne résidente, indispensable à la bonne santé de la peau, est constituée principalement de staphylocoques (*Staphylococcus epidermis* et *Staphylococcus hominis*), de corynebactéries, de propionibactéries Gram+ telles que *Propionibacterium acnes*, ainsi que d'une flore fongique principalement composée de *Pytosporum ovale.*

Les infections cutanées sont le plus souvent dues à la rupture de l'équilibre écologique de la flore résidente suite à la colonisation de la peau par des germes exogènes pathogènes ou à la prolifération anormale d'une souche endogène. Les germes pathogènes les plus connus sont *Pseudomonas aeruginosa* (Gram -) qui est responsable de petits boutons, de folliculites, de rougeurs et de prurit, *Candida albicans* pouvant provoquer des inflammations à la commissure des lèvres, candidoses cutanées, du prurit, des folliculites et des aphtes, *Staphylococcus aureus* pouvant provoquer des boutons, des folliculites, d'impétigo, et des furoncles, et *Streptococcus* du groupe A responsable d'impétigo.

Le document EP-315912 décrit l'utilisation de composés ayant une action anti-séborrhéique. Le document WO95/31956 décrit une émulsion antibactérienne constituée d'une huile et d'un ester de glycérol afin d'inactiver la bactérie *Helicobacter pylori*. Le document JP05/186328 décrit une composition pour nettoyer la peau à base d'une huile végétale et d'un alcool en présence d'un polysiloxane volatil. Le document DE 4330664 décrit une composition contenant au moins une huile végétale pour traiter des maladies comme le psoriasis ou la dermatite. Le document FR2768925 décrit une composition cosmétique comportant des extraits de lierre et de tournesol. Le document DE 19634956 décrit une composition pour son usage dans différentes maladies de la peau comprenant l'huile de germe de blé ou d'amande douce ou de camomille. Le document US 4172149 décrit une composition cosmétique comprenant des triglycérides d'acides gras pour son utilisation dans les cas de sécrétion excessive de sébum. Le document DE 4134137 décrit une lotion bioactive pour stimuler la croissance des cheveux et éviter la formation de pellicules. Le document FR2689011 décrit une préparation cosmétique pour combattre les lésions cutanées labiales à base d'huile végétale, de teinture végétale et d'huiles essentielles. Le document US 4451480 décrit une composition pour le traitement de l'acné, comprenant des ozonides d'huiles végétales. Le document CH688787 décrit des compositions destinées aux soins de la peau comprenant des huiles naturelles.

Pour combattre ces micro-organismes, il est courant d'utiliser des antibiotiques ou bactéricides. L'utilisation de ces composés pose cependant le problème de la non-spécificité d'action visant indifféremment la flore pathogène et la flore résidente, le problème du risque d'apparition de résistances bactériennes, ainsi que des problèmes de tolérance cutanée (irritations, allergies).

Il est également connu de réduire ou de prévenir la colonisation de surfaces telles que les dents, la peau et/ou des muqueuses, par des germes pathogènes en empêchant leur fixation sur ces supports. Les composés utilisés en tant qu'agents anti-adhésion décrits dans l'art antérieur sont des hydrates de carbone et des dérivés d'hydrates de carbone (WO 96/23 479, EP 380 084, US 5 002 759, US 4 859 656, WO 81/03 175, WO 93/14 773, WO 95/15 149, WO 95/07 084 et WO 95/17 898).

Or, la plupart des hydrates de carbone constituent une source de carbone pour des bactéries et champignons. Leur présence dans des compositions cosmétiques favorise par conséquent la prolifération microbienne et nécessite l'augmentation de la concentration en agents conservateurs (bactéricides ou bactériostatiques). Cet inconvénient annule ainsi le bénéfice de l'approche consistant à remplacer des composés antibiotiques ou bactéricides par des composés réduisant l'adhérence microbienne.

La demanderesse a trouvé de manière surprenante que des corps gras particuliers, exempts de motifs hydrates de carbone, permettaient de réduire significativement l'adhérence microbienne sur la peau et/ou les muqueuses et de prévenir ainsi la prolifération de germes potentiellement pathogènes en l'absence d'agents antibiotiques, bactéricides ou fongicides.

Ces corps gras, à la différence des hydrates de carbone qui se lient aux récepteurs des micro-organismes pour empêcher les liaisons aux glycolipides des cornéocytes, agissent sur les propriétés physico-chimiques de la surface de la peau et/ou des muqueuses, ces propriétés physico-chimiques faisant intervenir les interactions électrodynamiques dues aux forces de Van der Waals, les interactions acido-basiques selon Lewis et les interactions électrostatiques.

En outre, ces corps gras ne sont pas bactéricides. De ce fait, ils ne provoquent pas d'effets secondaires indésirables sur la peau et/ou les muqueuses.

Les corps gras selon l'invention, utilisés en tant que principes actifs, permettent de réduire ou d'empêcher l'adhésion d'un micro-organisme dont la charge globale de surface est négative ou positive en augmentant la charge respectivement négative ou positive de la peau, de manière à entraîner une répulsion entre la peau et/ou les muqueuses et le micro-organisme.

Les corps gras selon l'invention, utilisés en tant que principes actifs, permettent en outre de réduire ou d'empêcher l'adhésion d'un micro-organisme, en limitant le plus possible les interactions du type Van der Waals entre la peau et/ou les muqueuses et le micro-organisme, en favorisant les interactions répulsives de type acide-base selon Lewis et en limitant les interactions attractives de type acide-base selon Lewis entre le micro-organisme et la peau et/ou les muqueuses.

L'invention a donc pour objet l'utilisation en tant que principe actif, dans une composition cosmétique ou pour la préparation d'une composition pharmaceutique, d'une quantité efficace d'au moins un corps gras exempt de motifs hydrates de carbone, de température de fusion inférieure à 35°C et ayant une tension interfaciale comprise entre 6 et 27 mN/m, modifiant les propriétés physico-chimiques de la surface de la peau et/ou des muqueuses, en vue d'empêcher ou réduire l'adhésion des micro-organismes sur ces dernières.

Par empêcher ou réduire l'adhésion des micro-organismes, il faut entendre que le corps gras ou la composition le contenant peut être utilisé aussi bien à titre préventif, pour sa capacité à prévenir, totalement ou partiellement, l'adhésion des micro-organismes, qu'à titre curatif pour sa capacité à faciliter le détachement des micro-organismes.

En outre, ces corps gras sont tels que le logarithme décimal du nombre moyen de bactéries viables adhérant sur l'épiderme reconstruit, après un test de mise en contact dudit épiderme avec le composé testé pendant 2 heures à 37°C, est inférieur d'au moins 0,3, de préférence de 0,5 et plus préférentiellement de 1, à celui obtenu par un test réalisé avec de l'eau dans les mêmes conditions.

L'épiderme reconstruit utilisé dans le test indiqué ci-dessus est l'épiderme humain reconstitué, équivalent de la peau humaine, vendu par la société EPISKIN.

Ce test permet d'évaluer les modifications des propriétés physico-chimiques de surface de la peau et/ou des muqueuses, faisant intervenir les interactions électrodynamiques de Van der Waals, les interactions acido-basiques selon Lewis et les interactions électrostatiques.

Le protocole expérimental du test sera défini ci-dessous.

On utilise plus préférentiellement en tant que principe actif dans une composition cosmétique ou pour la préparation d'une composition pharmaceutique, une quantité efficace de corps gras exempts de motifs hydrates de carbone, de température de fusion inférieure à 35°C et ayant une tension interfaciale comprise entre 6 et 27 mN/m, choisis parmi :
- Les triglycérides d'acides gras généralement en C₈-C₃₀ comme les triglycérides d'acides caprylique/caprique et les huiles végétales telles que les huiles de germe de blé, de calendula, de ricin, d'olive, d'avocat, d'amande douce, d'arachide, de jojoba, de sésame, d'amande d'abricot, de tournesol, de macadamia, le beurre de karité.
- Les esters gras comportant une ou plusieurs chaînes linéaires ou ramifiées comportant notamment de 8 à 30 atomes de carbone, comme le laurate d'hexyle, l'oléate de décyle, le néopentanoate d'octyl dodécyle, le myristate d'isopropyle, l'isostéarate d'isopropyle, le stéarate d'isopropyle, l'adipate de dioctyle, l'isononanoate d'isononyle, le tartrate de di-alcools C₁₂-C₁₃ ramifiés, comme le produit vendu sous la dénomination Cosmacol ETI par la société Enichem, et les esters gras de chaînes linéaires ou ramifiées contenant une fonction glycéryle comme par exemple le palmitate d'octoxyglycéryle (ou palmitate d'éthyl-2-hexyl glycéryl éther) comme le produit commercialisé sous la dénomination Mexanyl GP par la société Chimex, le béhénate d'octoxyglycéryle (ou béhénate d'éthyl-2-hexyl glycéryl éther), le palmitate d'isopropyle et le Di-C12-13-alkylmalate comme le produit vendu sous la dénomination Cosmacol FM par la société Enichem.

On utilisera encore plus préférentiellement en tant que principe actif selon l'invention, l'huile de sésame, le palmitate d'octoxyglycéryle, le béhénate d'octoxyglycéryle, l'adipate de dioctyle, l'huile d'amande d'abricot, le tartrate de di-alcools C₁₂-C₁₃ ramifiés et/ou leurs mélanges

Selon l'invention, on utilise le corps gras ou la composition le contenant en application topique sur la peau et/ou les muqueuses.

L'adhésion de micro-organismes à la peau et/ou aux muqueuses a des conséquences qui vont du simple désagrément (l'odeur, les petits boutons) aux maladies plus ou moins graves.

Un des aspects de l'invention est donc de proposer l'utilisation d'un corps gras exempt de motifs hydrates de carbone, de température de fusion inférieure à 35°C et ayant une tension interfaciale comprise entre 6 et 27 mN/m, en tant que principe actif dans une composition cosmétique ou pour la préparation d'une composition pharmaceutique.

Particulièrement, l'invention a pour objet l'utilisation cosmétique en application topique d'au moins un corps gras, tel que défini ci-dessus, en tant que principe actif dans une composition cosmétique destinée à diminuer les mauvaises odeurs corporelles.

La flore microbienne de la surface de la peau est responsable d'un grand nombre de désordres.

La quantité de corps gras utilisable selon l'invention dépend bien évidemment de l'effet recherché et doit être une quantité efficace pour empêcher partiellement ou totalement l'adhésion des micro-organismes ou pour faciliter le détachement des micro-organismes.

A titre d'exemple, la quantité de corps gras utilisable selon l'invention peut aller par exemple de 0,1 à 100 % et de préférence de 0,5 à 50% et mieux de 5 à 25% du poids total de la composition.

Par milieu cosmétiquement acceptable, on entend un milieu compatible avec la peau, le cuir chevelu, les muqueuses, les ongles, et les cheveux.

Les compositions cosmétiques et pharmaceutiques utilisées selon l'invention peuvent se présenter sous toutes les formes galéniques normalement utilisées pour une application topique, en particulier sous forme de produits anhydres, liquides, pâteux ou solides tels que des lotions huileuses, des gels huileux, des onguents, ou sous forme d'émulsions huile-dans-eau ou eau-dans-huile ou multiple, ou d'une dispersion d'huile dans une phase aqueuse à l'aide de sphérules, ces sphérules pouvant être des nanoparticules polymériques telles que les nanosphères et les nanocapsules, ou mieux, des vésicules lipidiques de type ionique et/ou non-ionique.

Afin que les compositions de l'invention soient plus agréables à utiliser, plus douces à l'application, plus nourrissantes et plus émollientes, il est possible d'ajouter une phase grasse supplémentaire dans le milieu de ces compositions tout en s'assurant de l'efficacité anti-adhérente du mélange.

La phase grasse supplémentaire représente, de préférence, de 0 à 50 % en poids total de la composition.

Cette phase grasse supplémentaire peut comporter une ou plusieurs huiles choisies de préférence dans le groupe constitué par :
les silicones volatiles ou non-volatiles, linéaires, ramifiées ou cycliques, organo-modifiées ou non, hydrosolubles ou liposolubles,
   - les huiles minérales telles que l'huile de paraffine et de vaseline,
   - les huiles d'origine animale telles que le perhydrosqualène,
   - les huiles synthétiques telles que les isoparaffines,
   - les huiles fluorées et perfluorées,
   - les esters d'acides gras.

Elle peut aussi comporter comme matières grasses supplémentaires un ou plusieurs alcools gras, acides gras ou cires (paraffine, cire de polyéthylène, Carnauba, cire d'abeilles).

De façon connue, les compositions utilisées dans l'invention peuvent en outre contenir des adjuvants habituels dans le domaine cosmétique tels que des solvants, des gélifiants et/ou épaississants classiques hydrophile ou lipophiles; des actifs hydrophiles ou lipophiles; des conservateurs; des antioxydants; des parfums; des émulsionnants; des agents hydratants; des agents pigmentants; des dépigmentants; des agents kératolytiques; des vitamines; des émollients; des séquestrants; des tensio-actifs; des polymères; des agents alcalinisants ou acidifiants; des charges; des agents anti-radicaux libres; des céramides; des filtres solaires (notamment ultra-violets); des répulsifs pour insectes; des agents amincissants; des matières colorantes; des anti-pelliculaires.

Comme tensio-actifs, on peut citer par exemple les tensioactifs siliconés tels que les polydiméthicone copolyols et les alkyldiméthicone copolyols comme par exemple le produit vendu sous la dénomination Abil EM90 par la société Goldschmidt; les esters d'acides gras et de polyols tels que le PEG-7 glycéryl cocoate comme le produit vendu par la société COGNIS sous la dénomination Cetiol HE.

Comme solvants, on peut citer les solvants organiques hydrophiles, et par exemple des mono-alcools inférieurs, linéaires ou ramifiés, ayant de 1 à 8 atomes de carbone comme l'éthanol, le propanol, le butanol, l'isopropanol, l'isobutanol; des polyéthylèneglycols ayant de 6 à 80 oxydes d'éthylène; des polyols tels que le propylèneglycol, l'isoprène glycol, le butylèneglycol, le glycérol; les mono- ou dialkyles d'isosorbide dont les groupements alkyle ont de 1 à 5 atomes de carbone comme le diméthyl isosorbide; les éthers de glycol comme le diéthylène glycol mono-méthyle ou mono-éthyléther et les éthers de propylène glycol comme le dipropylène glycol méthyléther.

Les quantités de ces différents adjuvants sont celles classiquement utilisées dans les domaines considérés.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

Les compositions utilisées dans la présente invention peuvent être plus ou moins fluides et avoir l'aspect d'une crème blanche ou colorée, d'une pommade, d'un lait, d'une lotion, d'un sérum, d'une pâte, d'une mousse ou d'un solide.

Elles peuvent éventuellement être appliquées sur la peau sous forme d'aérosol.

Elles peuvent également se présenter sous forme solide, et par exemple sous forme de stick.

Elles peuvent être utilisées comme produit de soin, comme produit de nettoyage pour la peau ou les cheveux, comme produit solaire, en tant que produit de maquillage comme les fonds de teint, les rouges à lèvres, les mascaras, les fards, et/ou comme simple produit déodorant.

Le test d'anti-adhésion répond au protocole ci-dessous :

Avant l'adhésion bactérienne, l'épiderme reconstruit est mis en contact pendant 2 heures avec 25 mg du corps gras à tester à 37°C. On y ajoute alors 1 ml de suspension bactérienne de *Staphylococcus aureus* à une concentration de 10⁷ germes/ml dans du Tryptone sel. Après incubation 24 heures à 37°C, la suspension bactérienne est vidée et cinq rinçages sont réalisés par 1 ml d'eau distillée stérile. L'épiderme reconstruit détaché de son support est alors broyé à l'aide d'un robot dans 18 ml de Tryptone sel. On effectue une dilution décimale de cette suspension dans le Tryptone sel, on procède ensuite à un ensemencement de 1 ml de la dilution dans 15 ml de la gélose Trypticase Soja et à l'incubation pendant 24 heures à 37°C. On dénombre ensuite les cellules adhérentes et viables.

Ce test d'anti-adhésion permet d'évaluer l'efficacité de molécules seules ou de produits finis.

Avant le test d'anti-adhésion, on met en oeuvre le test de viabilité suivant :

Un mélange bactéries/produit à tester, dans le même rapport que dans le test anti-adhésion, est mis en contact 24 heures à 37°C. Le test peut nécessiter une incubation sous agitation pour éviter la mort des bactéries par manque d'oxygène, pour certains corps gras. Le dénombrement des germes est réalisé par dilution décimale dans du Tryptone sel et ensemencement au râteau de 100 µl sur de la gélose Trypticase Soja. Le comptage des colonies s'effectue après 24 heures d'incubation à 37°C.

L'essai de viabilité réalisé préalablement au test d'anti-adhésion permet d'écarter toute composante bactéricide des molécules ou des produits finis testés et de ne mettre en évidence que l'activité anti-adhésion.

Les exemples suivants présentent les résultats obtenus pour différents corps gras de tension interfaciale comprise entre 6 et 27 mN/m et de température de fusion inférieure à 35°C selon l'invention et une réalisation particulière de composition selon l'invention.

Ces exemples sont bien entendu donnés à titre illustratif et n'ont absolument pas de caractère limitatif.

### Exemples de corps gras utilisés selon l'invention :

Les résultats obtenus, pour les corps gras ici présentés, résultent de la mise en oeuvre du protocole ci-dessus détaillé.

Les chiffres présentés en face du corps gras correspondent à la diminution du logarithme décimal du nombre moyen de *Staphylococcus aureus* viable adhérent sur l'épiderme reconstruit après traitement par le corps gras dans les conditions définies par le test précédent par rapport au logarithme décimal du nombre moyen de *Staphylococcus aureus* viable adhérent sur l'épiderme reconstruit après traitement à l'eau dans les mêmes conditions.

| **Huile** | **Diminution du log/témoin** |
|---|---|
| Huile d'olive | 3,25 |
| Huile d'amande douce | 1,34 |
| Huile de sésame | 1,92 |
| Huile d'amande d'abricot | 0,81 |
| Huile de tournesol | 1,4 |
| Adipate de dioctyle | 0,9 |
| Tartrate de di-alcools C₁₂-C₁₃ ramifiés | 2,31 |
| Palmitate d'octoxyglycérile | 1,85 |
| Béhénarate d'octoxyglycérile | 2,59 |
| Palmitate d'isopropyle | 1,07 |
| Di-C12-13-alkyl malate | 3,68 |

### Exemple de l'importance de l'utilisation de corps gras de tension interfaciale comprise entre 6 et 27 mN/m :

Les chiffres présentés en face du corps gras correspondent à la valeur du logarithme décimal du nombre moyen de *Staphylococcus aureus* viable adhérent sur l'épiderme reconstruit après traitement par le corps gras dans les conditions définies par le test précédent par rapport au logarithme décimal du nombre moyen de *Staphylococcus aureus* viable adhérent sur l'épiderme reconstruit après traitement à l'eau dans les mêmes conditions.

| **Huile** | **Tension interfaciale** | **Log mesuré** |
|---|---|---|
| Squalane | 46mN/m | Augmentation de 0,27/témoin |
| Polyisobutène hydrogéné | 40mN/m | Sans effet |

### Exemple de composition utilisée selon l'invention :

| Emulsion E/H pour le soin du visage | |
|---|---|
| Poly méthylcétyl diméthyl méthylsiloxane | |
| Oxyéthyléné (ABIL EM 90 ® de GOLDSCHMIDT) | 3 % |
| Palmitate d'éthyl-2-hexyl glycéryl éther | |
| (MEXANYL GP ® de CHIMEX) | 10 % |
| Tartrate de di-alcools C₁₂-C₁₃ ramifiés | |
| (COSMACOL ETI ® de ENICHEM) | 10 % |
| Cocoate de glycéryle oxyéthyléné (7 OE) | |
| (CETIOL HE ® de GOGNIS) | 3 % |
| Condensat d'oxyde d'éthylène, d'oxyde de propylène et d'oxyde d'éthylène (PM : 8350) (75 OE/30 OP/75 OE) (LUTROL F 68 ® de BASF) | 3 % |
| Eau QSP | 100 |
| Anti-oxydant | qs |
| Parfum | qs |

Après traitement par la composition ci-dessus, dans les conditions définies par le test précédent, on observe une diminution du logarithme décimal du nombre moyen de *Staphylococcus aureus* viable adhérent sur l'épiderme reconstruit par rapport au logarithme décimal du nombre moyen de *Staphylococcus aureus* viable adhérent sur l'épiderme reconstruit après traitement à l'eau dans les mêmes conditions de 3,96.

## Revendications

1. Utilisation en tant que principe actif, dans une composition cosmétique ou pour la préparation d'une composition pharmaceutique, d'une quantité efficace d'au moins un corps gras exempt de motifs hydrates de carbone, de température de fusion inférieure à 35°C et ayant une tension interfaciale comprise entre 6 et 27 mN/m, modifiant les propriétés physico-chimiques de la surface de la peau et/ou des muqueuses, en vue d'empêcher ou réduire l'adhésion des micro-organismes sur ces dernières.

2. Utilisation selon la revendication 1, **caractérisée en ce que** le principe actif est choisi parmi les triglycérides d'acides gras, les huiles végétales et les esters gras comportant une ou plusieurs chaînes linéaires ou ramifiées, comportant 8 à 30 atomes de carbone.

3. Utilisation selon l'une quelconque des revendications 1 ou 2, **caractérisée en ce que** le principe actif est choisi parmi les triglycérides d'acides caprylique/caprique, l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia, le beurre de karité, le laurate d'hexyle, l'oléate de décyle, le néopentanoate d'octyl dodécyle, le myristate d'isopropyle, l'isostéarate d'isopropyle, le stéarate d'isopropyle, l'adipate de dioctyle, l'isononanoate d'isononyle, le tartrate de di-alcools C₁₂-C₁₃ ramifiés, le palmitate d'octoxyglycéryle, le béhénate d'octoxyglycéryle et/ou leurs mélanges, le palmitate d'isopropyle et le Di-C12-13-alkylmalate comme le produit vendu sous la dénomination Cosmacol FM par la société Enichem.

4. Utilisation selon l'une quelconque des revendications 1 à 3, **caractérisée en ce que** le principe actif est choisi parmi l'huile de sésame, le beurre de karité, le palmitate d'octoxyglycéryle, le béhénate d'octoxyglycéryle, l'adipate de dioctyle, l'huile d'amande d'abricot, le tartrate de di-alcools C₁₂-C₁₃ ramifiés et/ou leurs mélanges, le palmitate d'isopropyle et le Di-C12-13-alkylmalate comme le produit vendu sous la dénomination Cosmacol FM par la société Enichem.

5. Utilisation selon l'une quelconque des revendications 1 à 4, **caractérisée en ce que** le principe actif est présent en une quantité allant de 0,1% à 100% du poids total de la composition.

6. Utilisation selon la revendication 5, **caractérisée en ce que** le principe actif est présent en une quantité allant de 0,5 % à 50 % du poids total de la composition.

7. Utilisation selon l'une quelconque des revendications 5 ou 6, **caractérisée en ce que** le principe actif est présent en une quantité allant de 5 % à 25 % du poids total de la composition.

8. Utilisation selon l'une quelconque des revendications 1 à 7, **caractérisée en ce que** la composition se présente sous la forme d'une lotion, d'un gel, d'un sérum, d'une émulsion ou d'une dispersion de vésicules lipidiques.

9. Utilisation cosmétique en application topique d'au moins un corps gras exempt de motifs hydrates de carbone, de température de fusion inférieure à 35°C et ayant une tension interfaciale comprise entre 6 et 27 mN/m en tant que principe actif dans une composition cosmétique destinée à diminuer les mauvaises odeurs corporelles.

## Patentansprüche

1. Verwendung einer wirksamen Menge mindestens einer Fettsubstanz, die keine Kohlenhydrateinheiten aufweist, eine Schmelztemperatur unter 35 °C und eine Grenzflächenspannung von 6 bis 27 mN/m besitzt und die physikalischchemischen Eigenschaften der Oberfläche der Haut und/oder der Schleimhäute verändert, in einer kosmetischen Zusammensetzung oder für die Herstellung einer pharmazeutischen Zusammensetzung, um das Anhaften von Mikroorganismen auf der Haut und/oder den Schleimhäuten zu verhindern oder zu vermindern.

2. Verwendung nach Anspruch 1, **dadurch gekennzeichnet, dass** der Wirkstoff unter den Triglyceriden von Fettsäuren, pflanzlichen Ölen und Fettsäureestern, die eine oder mehrere geradkettige oder verzweigte Gruppen mit insbesondere 8 bis 30 Kohlenstoffatomen aufweisen, ausgewählt ist.

3. Verwendung nach einem der Ansprüche 1 oder 2, **dadurch gekennzeichnet, dass** der Wirkstoff unter Triglyceriden von Capryl/caprinsäure, Weizenkeimöl, Calendulaöl, Ricinusöl, Olivenöl, Avocadoöl, Süßmandelöl, Erdnussöl, Jojobaöl, Sesamöl, Aprikosenkernöl, Sonnenblumenöl, Macadamiaöl, Sheabutter, Hexyllaurat, Decyloleat, Octyldodecylneopentanoat, Isopropylmyristat, Isopropylisostearat, Isopropylstearat, Dioctyladipat, Isononylisononanoat, dem Tartrat von verzweigten C₁₂₋₁₃-Dialkoholen, Octoxyglycerylpalmitat, Octoxyglycerylbehenat und/oder deren Gemischen, Isopropylpalmitat und Di-C₁₂₋₁₃-Alkylmalat, wie dem Produkt mit der Bezeichnung Cosmacol FM der Firma Enichem, ausgewählt ist.

4. Verwendung nach einem der Ansprüche 1 bis 3, **dadurch gekennzeichnet, dass** der Wirkstoff unter Sesamöl, Sheabutter, Octoxyglycerylpalmitat, Octoxyglycerylbehenat, Dioctyladipat, Aprikosenkernöl, dem Tartrat von verzweigten C₁₂₋₁₃-Dialkoholen und/oder deren Gemischen, Isopropylpalmitat und Di-C₁₂₋₁₃-Alkylmalat, wie dem Produkt mit der Bezeichnung Cosmacol FM der Firma Enichem, ausgewählt ist.

5. Verwendung nach einem der Ansprüche 1 bis 4, **dadurch gekennzeichnet, dass** der Wirkstoff in einer Menge von 0,1 bis 100 % des Gesamtgewichts der Zusammensetzung vorliegt.

6. Verwendung nach Anspruch 5, **dadurch gekennzeichnet, dass** der Wirkstoff in einer Menge von 0,5 bis 50 % des Gesamtgewichts der Zusammensetzung vorliegt.

7. Verwendung nach einem der Ansprüche 5 oder 6, **dadurch gekennzeichnet, dass** der Wirkstoff in einer Menge von 5 bis 25 % des Gesamtgewichts der Zusammensetzung vorliegt.

8. Verwendung nach einem der Ansprüche 1 bis 7, **dadurch gekennzeichnet, dass** die Zusammensetzung als Lotion, Gel, Serum, Emulsion oder Dispersion von Lipidvesikeln vorliegt.

9. Kosmetische Verwendung mindestens einer Fettsubstanz, die keine Kohlenhydrateinheiten aufweist, eine Schmelztemperatur unter 35 °C und eine Grenzflächenspannung von 6 bis 27 mN/m besitzt als Wirkstoff in einer kosmetischen Zusammensetzung, die dazu vorgesehen ist, schlechte Körpergerüche zu vermindern.

## Claims

1. Use, as active ingredient, in a cosmetic composition or for the preparation of a pharmaceutical composition, of an effective quantity of at least one fatty substance free of carbohydrate units, having a melting point of less than 35°C and having an interfacial tension of between 6 and 27 mN/m, modifying the physicochemical properties of the surface of the skin and/or of the mucous membranes so as to prevent or reduce the adhesion of microorganisms to the latter.

2. Use according to Claim 1, **characterized in that** the active ingredient is chosen from fatty acid triglycerides, vegetable oils and fatty esters comprising one or more linear or branched chains, comprising 8 to 30 carbon atoms.

3. Use according to either of Claims 1 and 2, **characterized in that** the active ingredient is chosen from caprylic/capric acid triglycerides, wheatgerm oil, calendula oil, castor oil, olive oil, avocado oil, sweet almond oil, groundnut oil, jojoba oil, sesame oil, apricot oil, sunflower oil, macadamia oil, shea butter, hexyl laurate, decyl oleate, octyl dodecyl neopentanoate, isopropyl myristate, isopropyl isostearate, isopropyl stearate, dioctyl adipate, isononyl isononanoate, tartrate of branched C₁₂-C₁₃ dialcohols, octoxyglyceryl palmitate, octoxyglyceryl behenate and/or mixtures thereof, isopropyl palmitate and di(C₁₂-C₁₃ alkyl) malate such as the product sold under the name Cosmacol FM by the company Enichem.

4. Use according to any one of Claims 1 to 3, **characterized in that** the active ingredient is chosen from sesame oil, shea butter, octoxyglyceryl palmitate, octoxyglyceryl behenate, dioctyl adipate, apricot stone oil, tartrate of branched C₁₂-C₁₃ dialcohols and/or mixtures thereof, isopropyl palmitate and di(C₁₂-C₁₃ alkyl) malate such as the product sold under the name Cosmacol FM by Enichem.

5. Use according to any one of Claims 1 to 4, **characterized in that** the active ingredient is present in a quantity ranging from 0.1% to 100% of the total weight of the composition.

6. Use according to Claim 5, **characterized in that** the active ingredient is present in a quantity ranging from 0.5% to 50% of the total weight of the composition.

7. Use according to either of Claims 5 and 6, **characterized in that** the active ingredient is present in a quantity ranging from 5% to 25% of the total weight of the composition.

8. Use according to any one of Claims 1 to 7, **characterized in that** the composition is provided in the form of a lotion, a gel, a serum, an emulsion or a dispersion of lipid vesicles.

9. Cosmetic use by topical application of at least one fatty substance free of carbohydrate units, having a melting point of less than 35°C and having an interfacial tension of between 6 and 27 mN/m as active ingredient in a cosmetic composition intended to reduce bad body odours.
